Europäisches Patentamt

European Patent Office

Office européen des brevets

(11) Publication number: **0 109 560**
A2

# (12) EUROPEAN PATENT APPLICATION

(21) Application number: 83110472.4

(22) Date of filing: 20.10.83

(51) Int. Cl.³: **C 12 N 15/00**
**C 12 P 21/02**

(30) Priority: 20.10.82 JP 184293/82

(43) Date of publication of application:
30.05.84 Bulletin 84/22

(84) Designated Contracting States:
AT BE CH DE FR GB IT LI LU NL SE

(71) Applicant: SUNTORY KABUSHIKI KAISHA, also known
as SUNTORY LTD.
1-40, Dojimahama 2-chome
Kita-ku Osaka(JP)

(72) Inventor: Oshima, Takehiro
17-10-383, Besshohonmachi
Takatsuki-shi Osaka(JP)

(72) Inventor: Arima, Kenji
6-A-1106, Sawaraginishi 1-chome
Ibaraki-shi Osaka(JP)

(72) Inventor: Tanaka, Shoji
8-46, Minamikanenden-cho 1-chome
Suita-shi Osaka(JP)

(72) Inventor: Nakazato, Hiroshi
13-E-505, Misawa-cho
Ibaraki-shi Osaka(JP)

(74) Representative: Hansen, Bernd, Dr.rer.nat. et al,
Hoffmann, Eitle & Partner Patentanwälte Arabellastrasse
4
D-8000 München 81(DE)

(54) Yeast plasmid vector capable of induced expressing and method of using said plasmid.

(57) A yeast vector for producing a useful peptide under the
control of the promoter of PH05 gene which is a gene
controlling a repressible yeast acid phosphatase, a process
of preparing the same and a process for producing the useful
peptide in high yield by culturing the yeast that has been
transformed by said vector are disclosed.

Croydon Printing Company Ltd.

-1-

# YEAST PLASMID VECTOR CAPABLE OF INDUCED EXPRESSING AND METHOD OF USING SAID PLASMID

The present invention relates to a yeast vector for producing a useful peptide under the control of the promoter of PHO5 gene (a gene controlling a repressible yeast acid phosphatase). The invention also relates to a process for producing the useful peptide in high yield by culturing yeast that has been transformed by said vector.

A repressible yeast acid phosphatase is an enzyme that is controlled by the concentration of phosphate, and the production of this enzyme can be induced by reducing the concentration of the phosphoric acid. At least two types of the repressible yeast acid phosphatase have been reported, and one of them is encoded by a PHO5 gene. The present inventors have succeeded in expressing a useful peptide under the control of the PHO5 gene by using a promoter for that gene.

The recent development of the technology of genetic engineering is so remarkable that today, pharmaceutically useful peptides such as somatostatin, insulin, growth hormones, interferons (alpha, beta and gamma), influenza viruses, hepatitis B viral protein, thymosin alpha 1, beta-endorphin, alpha-neoendorphin, secretin, urokinase and plaminogen activating materials can be synthesized in microorganisms or animal cells.

In most cases, these materials are produced by using procaryotic E. coli as host cells. Recently, the use of eukaryotic yeast cells is drawing the attention of many researchers for such reasons as easy cultivation of yeast, rapid cell division, low hazard, and advanced analysis of the genetic and biochemical aspects of Saccharomyces yeast.

In order to express genes for useful peptides (including exogenous peptide genes and genes encoding endogenous peptides produced only in small amounts in the yeast), the presence of a promoter region adapted to the specific host cells is required, and the efficiency of the expressing of peptide genes greatly depends on the promoter used. Therefore, intensive efforts have been made to develop expressing plasmid vectors using various promoters.

However, the vectors so far reported for expressing exogenous genes using yeast cells as the host are not highly efficient in expressing the desired exogenous gene as compared with conventional vectors using E. coli cells as the host.

Noting the use of the promoter for an operon encoding the repressible yeast acid phosphatase (PHO5), the present inventors have made studies to construct vectors capable of expressing the target peptide gene in high efficiency. As a result, the inventors found that this promoter was very useful in the production of exogenous or endogenous peptides in the yeast. We prepared a yeast vector having the desired peptide gene inserted at a point downstream of the promoter, and used the so prepared vector in transforming the yeast. By analyzing the separated yeast cells, we could confirm that they had been transformed by the said vector. This has lead to the accomplishment of the present invention.

The target peptide genes used in the present invention include chemically synthesized genes, complementary DNA (cDNA) prepared from mRNA by reverse transcriptase, and genes isolated from chromosomes by a suitable treatment.

Fig. 1 is a restriction map showing the recognition sides for restriction enzymes acting on a BamHI fragment containing the PHO5 gene.

Figs. 2 (a) to (d) show the entire base sequence of the PHO5 gene.

Fig. 3 shows the procedure for constructing a yeast vector bearing the PHO5 gene.

Fig. 4 shows the procedure for constructing a yeast vector beairng the PHO5 gene which expresses a gene for the target peptide.

Fig. 5 shows the procedure for inserting αNE (alpha-neo-endorphin) gene into a PHO5-bearing vector.

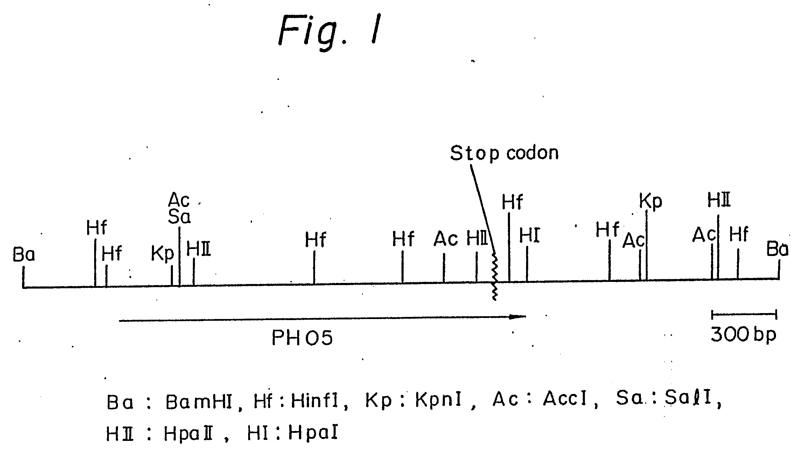

The yeast vector bearing the PHO5 gene according to the present invention is prepared by the following procedure. Chromosomal DNA of Saccharomyces yeast is digested into two fragments by restriction enzyme BamHI, and the PHO5 gene

used in the present invention is located on a DNA fragment having a length of about 3 kb. This PHO5 gene encodes a protein composed of 450 amino acid residues having a signal peptide made of 17 amino acid residues as counted from the amino acid sequence at N-terminal of the purified enzyme. The recognition sequences for various restriction enzymes working on the BamHI fragment containing the PHO5 gene are shown in the restriction map of Fig. 1, wherein the respective symbols denote the following: Ba: BamHI, Hf: HinfI, Kp: KpnI, Ac: AccI, Sa: SalI, HII: HpaII and HI: HpaI.

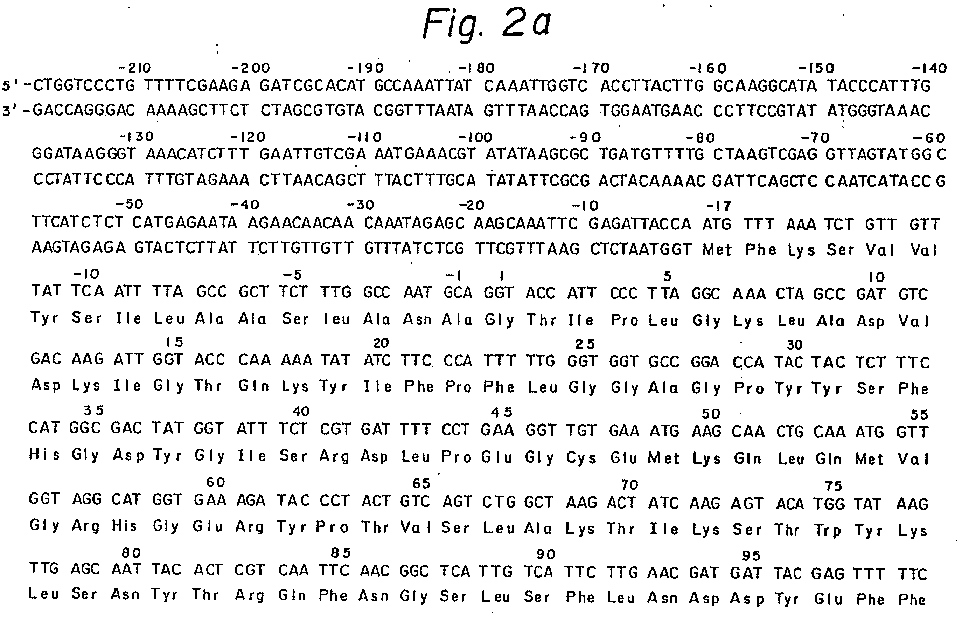

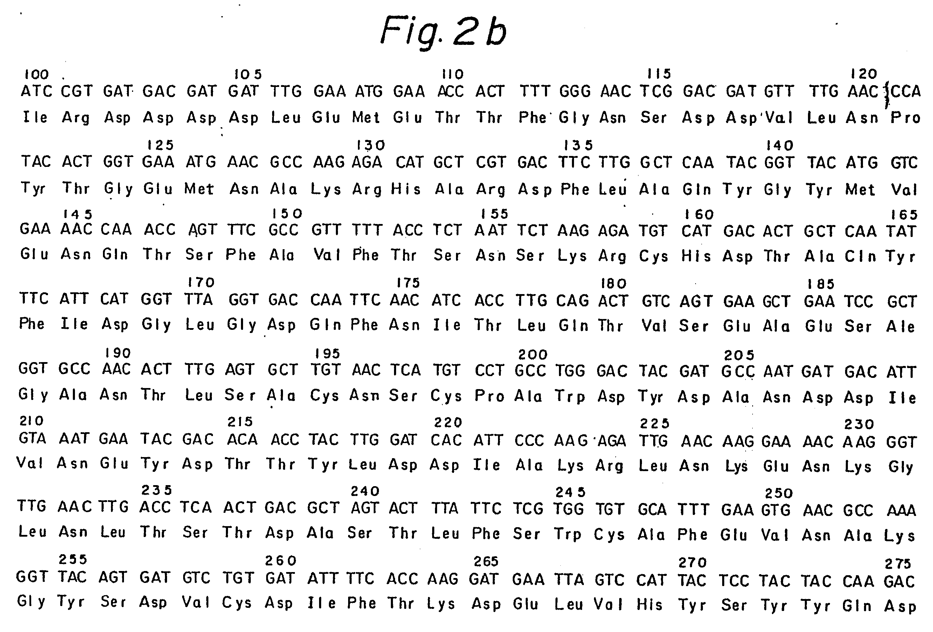

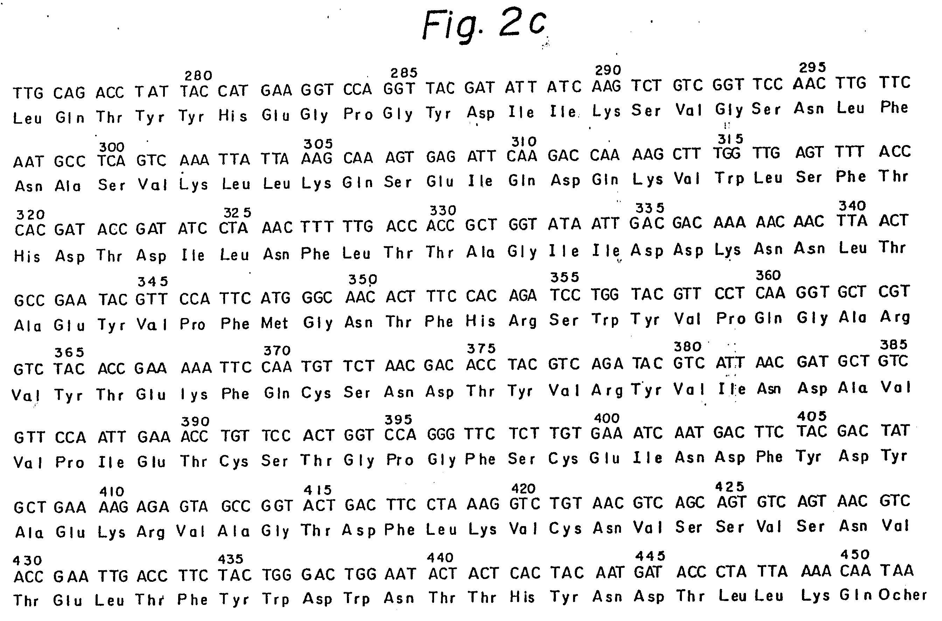

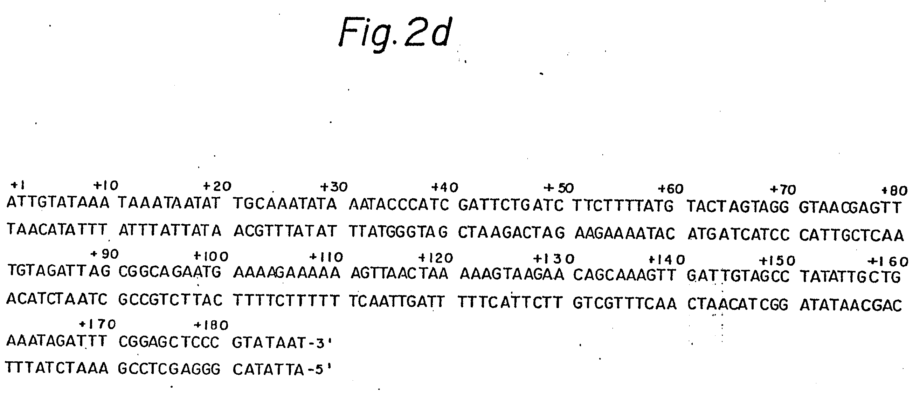

The entire base sequence of the BamHI fragment containing the PHO5 gene is shown in Fig. 2. This fragment is inserted into a suitable vector bearing a replicon and a selective marker, such as pYE207 which is a plasmid having 2 μm replicon and $Leu2^+$ gene bound to pBR322. The target peptide gene is inserted into the plasmid at a point downstream of the PHO5 gene promoter, thereby preparing a yeast vector bearing PHO5 promoter, target peptide gene and selective marker. If the target peptide has a low molecular weight such as in the case of alpha-neoendorphin (αNE), it must be produced as a chimeric protein (PHO5-αNE) by inserting the target peptide gene in the PHO5 structural gene at a suitable location. However, if the target peptide has a relatively high molecular weight such as in the case of an interferon, it can be directly produced by attaching a gene coding for the peptide to the PHO5 promoter with a suitable base sequence being interposed. Therefore, the PHO5 promoter can be extensively used not only to inducively express endogenous or exogeneous peptide genes but also to produce the target peptide.

By using the αNE gene in the vector of the present invention, alpha-neoendorphin can be expressed as a PHO5-αNE chimeric protein and the αNE molecules can be separted from the chimeric protein by cleaving it using cyanogen bromide or other conventional techniques. Radioimmunoassay (RIA) showed that the productivity of this method was about $10^6$ αNE molecules per cell.

The present invention is described in greater detail by reference to the following non-limiting example.. In the example, the αNE gene is used as the target peptide gene, but it should be noted that the scope of the present invention is by no means limited to the production of αNE.

Example

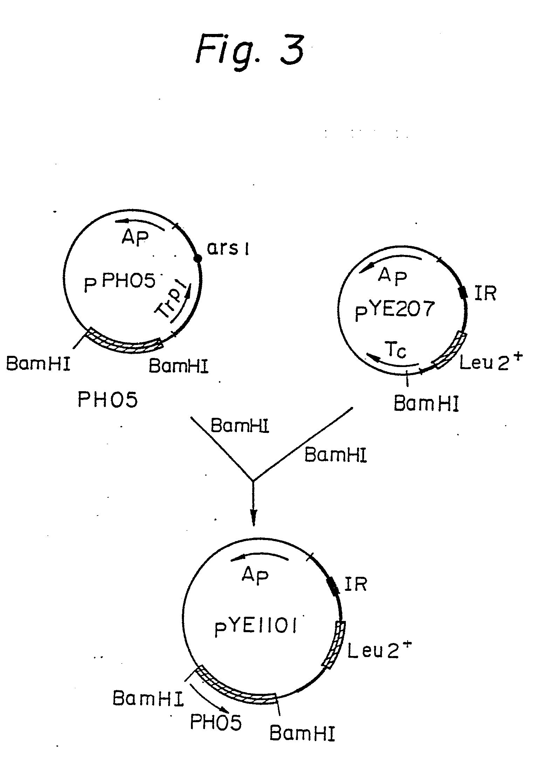

1. Preparation of yeast plasmid pYE1101 bearing PHO5 gene (see Fig. 3)

The repressible yeast acid phosphatase (PHO5) is located on a DNA fragment (ca. 3 kb) obtained by cleaving the chromosome DNA of Saccharomyces yeast with BamHI. When pPHO5 prepared by cloning this DNA fragment at BamHI site of YRp7 is introduced into a pho5$^-$ yeast host, the host is transformed to exhibit PHO$^+$ phenotype. Replication of the plasmid pPHO5 is controlled by a chromosomal replicon and is unstable in yeast cells. Therefore, we cleaved this plasmid with BamHI, selected a DNA fragment (ca. 3 kb) bearing the PHO5 gene, and inserted this fragment at BamHI site of pYE207 (plasmid having 2 μm replicon and Leu$^{2+}$ gene bound to pBR322) so as to prepare pYE1101. This plasmid remained stable in yeast cells and the acid phosphatase was produced in the presence of a low concentration of phosphate.

The results of transformation of yeast cells with pYE 1101 are shown in Table 1, from which one can see that pYE 1101 bears the PHO5 gene and that this gene is expressed in the yeast.

## Table 1

### Productivity of acid phosphatase in yeast transformed by plasmid pYE1101

| Medium | Plasmid | Cell No. $(OD_{660})$ | Activity of acidic phosphatase | |
|--------|---------|------------------------|----------------------------------|----------------------------------|
| | | | $OD_{420}$ (ml/min) | $OD_{420}/OD_{660}$ |
| +Pi* | — | 16.5 | 0.005 | 0.0003 |
| | pYE1101 | 14.2 | 0.450 | 0.032 |
| -Pi** | — | 10.0 | 0.250 | 0.025 |
| | pYE1101 | 6.25 | 2.800 | 0.448 |

* +P: Non-inducible medium; **-P: Inducible medium

Saccharomyces cerevisiae XP3-7A cells that were transformed by plasmid pYE1101 were named Saccharomyces cerevisiae SBM 311 and deposited with the Fermentation Research Institute, Japan (deposit No. FERM P-6764; International Deposition Acceptance No. FERM BP-383 under Butapest Treaty).

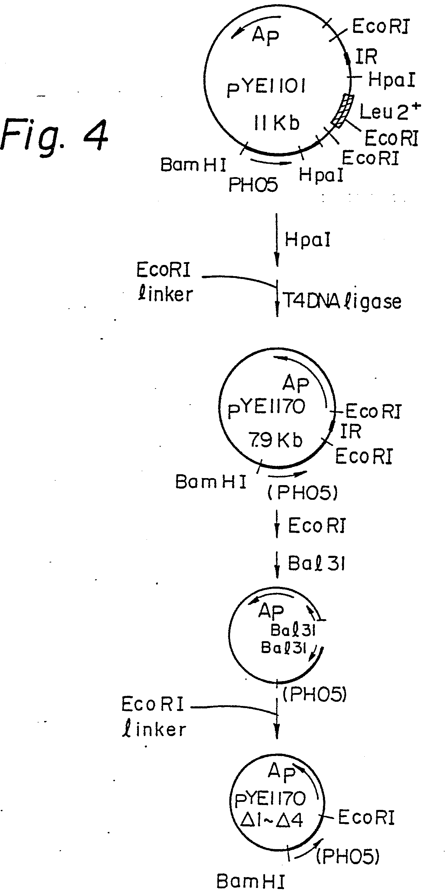

2. Preparation of PHO5 vector for expressing the target peptide gene (see Fig. 4)

Plasmid pYE1101 (10 µg, 11 kb) was cleaved with HpaI (30 units) by heating in TA buffer (33 mM tris-acetic acid buffered at pH 7.9, 66 mM potassium acetate, 10 mM magnesium acetate and 0.5 mM DTT) at 37°C for 1.5 hours. EcoRI linker (5 µg; 5'-GGAATTCC-3') was incubated in a polynucleotide kinase buffer (50 mM tris-HCl buffered at pH 7.5, 10 mM MgCl$_2$) at 37°C for 45 minutes in the presence of ATP (25 nmol) and polynucleotide kinase (10 units) so as to phosphorylate the 5'-terminals.

The 5 µg HpaI fragments (7.9 kb and 3.1 kb) of pYE1101 and phosphorylated EcoRI linker (1 µg) were dissolved in 20 1 of a ligase buffer and incubated at 15°C for 18 hours in the presence of T4 DNA ligase (5 units). The reaction solution (10 µl) was introduced into E. coli JA221 that had been treated with 0.3 ml of CaCl$_2$. Plasmid DNA was separated from the amcillin-resistant transformants in the conventional manner and analyzed to confirm the construction of a plasmid pYE1170 having EcoRI linker inserted to HpaI site of a 7.9 kb fragment of pYE1101. This plasmid (5 µg) was cleaved into two fragments (6.5 kb and 1.4 kb) with EcoRI (30 units) by incubating in TA buffer at 37°C for 1 hour. By subsequent heating at 65°C for 5 minutes, EcoRI was deactivated, and thereafter, the DNA was precipitated by adding ethanol twice the volume of the reaction solution. The DNA precipitate was dissolved in a buffer solution (50 1) consisting of 20 mM tris-HCl (pH 8.1), 100 mM NaCl and 12 mM CaCl$_2$ at 30°C for 3 minutes in the presence of nuclease BAL-31 (0.5 unit). By this treatment, 200-400 bp of DNA was broken down. After inactivating BAL-31 by heating the solution at 65°C, the DNA was precipitated by adding ethanol twice the volume of the solution. The DNA precipitate was dissolved in T4 ligase buffer (20 µl), added with phosphorylated EcoRI linker (1 µg) and subjected to ligation at 15°C for 18 hours in the presence of T4 DNA ligase (5 units). The reaction solution (10 µl) was introduced into E. coli JA221 that has been treated with 0.3 ml of $CaCl_2$.

Plasmid DNA was separated from the ampicillin-resistant transformants by the conventional method and analyzed to confirm the construction of plasmid pYE1170 Δ1-Δ4 about 6.2 kb long and having a single EcoRI cleavage site.

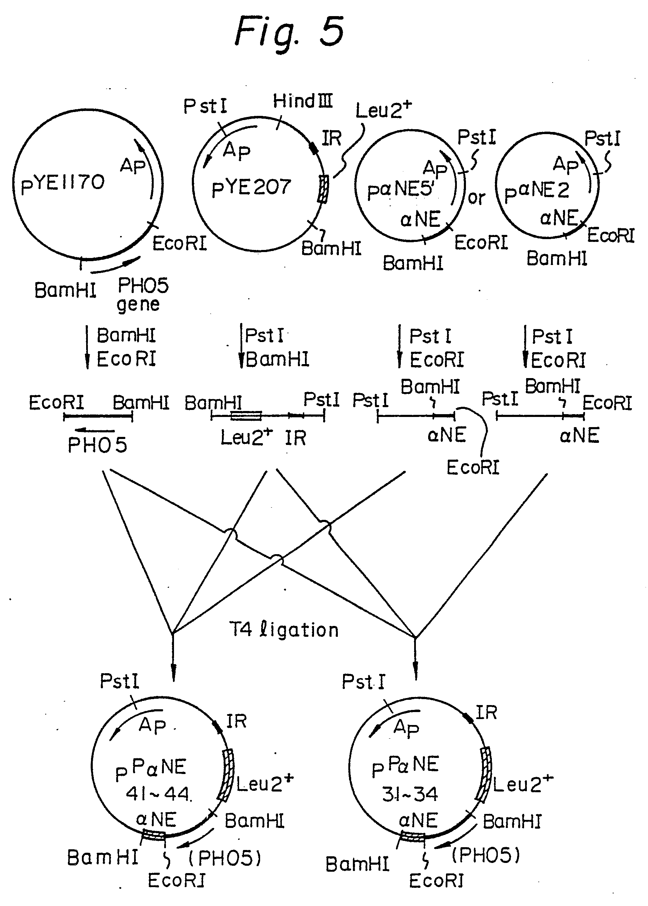

3. Insertion of αNE gene into PHO5 vector (see Fig. 5)

The αNE gene was prepared from pαNE2 (Nucleic Acids Res., 10: 1741-1754, 1982) and pαNE5' (this plasmid had the base sequence of 5'-AATTCTA ATG (Met) ---3' upstream of the methionine codon of the αNE gene in pαNE2 and was to be cut by EcoRI at a site 2 base pairs far than in pαNE2, thus causing offsetting of the reading frame by 2 base pairs at the EcoRI site as compared with the case of pαNE2). An outline for the method of inserting the αNE gene into the PHO5 vector is shown in Fig. 5.

Plasmid pYE1170 Δ1-Δ4 (5 µg) was cleaved with BamHI (10 units) and EcoRI (10 units) by incubating in a TA buffer at 37°C for 1 hour. Plasmid pYE207 (5 µg) was cleaved with PstI (10 units) and BamHI (10 units) by incubating in TA buffer at 37°C for 1 hour. Two other plasmids pαNE2 and pαNE5' were each cleaved with PstI (10 units) and EcoRI (10 units) by incubating in TA buffer at 37°C for 1 hour. The respective reaction solutions were subjected to agarose gel electrophoresis to separate the desired DNA fractions: a fragment containing PHO5 gene was separated from pYE1170 Δ1-Δ4; a fragment containing $Leu2^+$ gene was separated from pYE207; and a fragment containing the αNE gene was separated from each of pαNE2 and pαNE5'. The respective DNA fragments were dissolved in T4 DNA ligase buffer (20 µl) and treated with T4 DNA ligase (2 units) at 15°C for 18 hours. For example, fragments from pYE1170 1, pYE207 and pαNE2 were ligated to obtain plasmid pPαNE31, whereas

fragments from pYE1170 Δ1, pYE207 and pαNE5' were ligated to obtain plasmid pPαNE41. Each of the ligation solutions (10 μl) was introduced into E. coli JA221 that had been treated with 0.3 ml of CaCl$_2$.

Plasmid DNA was separated from the ampicillin-resistant transformants by the conventional technique and analyzed to conform the construction of eight plasmids pPαNE31, 32, 33 and 34, as well as pPαNE 41, 42, 43 and 44, each having the PHO gene ligated to the αNE gene (see Fig. 4). These eight plasmids were introduced into a yeast strain, Saccharomyces XP3-7A (a leu2 his4 trp1 pho5). The cells transformed by plasmid pPαNE42 were named Saccharomyces cerevisiae SBM 314 and have been deposited with the Fermentation Research Institute, Japan (deposit No. FERM P-6765; International Deposition Acceptance No. FERM BP-384 under the Budapest Treaty).

The respective transformants were shake-cultured in -PiYPD media (low phosphate media prepared by adding 10 ml of 1M MgSO$_4$ and 10 ml of ammonium hydroxyde (28%) to 1,000 ml of a solution containing 1% yeast extract and 2% polypeptone, filtering the resulting precipitate, and adding glucose to obtain a concentration of 0.2%) and +PiYPD media (high phosphate media prepared by adding 0.2% KH$_2$PO$_4$ to the -PiYPD media) at 30°C for 24 hours, and thereafter, the cells were recovered by centrifugation. The cells recovered from 1 ml of the culture were mixed with 0.5 ml of cold acetone and the mixture was left to stand at -20°C for 1-24 hours, and freeze-dried. The dried cells were suspended in 70% formic acid containing cyanogen bromide (5 mg/ml) and the suspension was left to stand in a dark place for 24 hours. The suspension was freeze-dried and αNE was eluted with 0.1 N acetic acid (0.1 ml). The eluted αNE was neutralized with 0.1 ml of 1.3M tris-HCl (pH 8.0) and subjected to radioimmunoassay (RIA) according to the method described in N. Minamino et al., BBRC, vol. 102, 226 (1981). The result was that only the yeast cells having a plasmid pPαNE41, 42 or 43 showed the αNE activity, and the cells having the other plasmids showed no αNE activity probably

because the αNE gene binding site did not have good matching of the reading frame. The productivity of αNE in yeast cells with pPαNE41, 42 and 43 that were cultured in -PiYPD and +PiYPD media is shown in Table 2 in comparison with the data for pYE1101. The table clearly shows that the productivity of NE greatly depends on the concentration of phosphate in the medium and is controlled by the PHO5 gene.

The productivity of αNE in the low-phosphate medium was about 200 times as high as that achieved with the high-phosphate medium, and by using the PHO5 promoter, about one million αNE molecules were produced per cell in the cultivation with the -PiYPD medium. The acid phosphatase in the yeast cells having the plasmids constructed by the present invention has the same level of activity as that of the yeast cells free from these plasmids.

<u>Table 2</u>

<u>Productivity of αNE in the presence of PHO5 promoter</u>

| Medium | Plasmid | Cell No. | αNE | |
|---|---|---|---|---|
| | | cells/ml | ng/ml | molecules/cell |
| +Pi | pYE1101 | $5.2 \times 10^7$ | 0 | — |
| | pPαNE41 | $5.0 \times 10^7$ | 0.8 | $8.0 \times 10^3$ |
| | pPαNE42 | $4.9 \times 10^7$ | 0.5 | $5.1 \times 10^3$ |
| | pPαNE43 | $4.8 \times 10^7$ | 0.5 | $5.2 \times 10^3$ |
| -Pi | pYE1101 | $3.0 \times 10^7$ | <0.18 | — |
| | pPαNE41 | $2.0 \times 10^7$ | 46.3 | $12. \times 10^6$ |
| | pPαNE42 | $2.2 \times 10^7$ | 50.0 | $1.1 \times 10^6$ |
| | pPαNE43 | $1.9 \times 10^7$ | 70.0 | $1.8 \times 10^6$ |

In order to check the site of binding between the PHO5 gene and αNE gene, plasmid DNA was separated from pPαNE41, 42 and 43 by digestion with EcoRI, and the base sequence of that DNA was determined according to the method of Maxam and Gilbert. The result was as follows: in pPαNE41, the 387th proline codon was bound to the αNE gene with a linker (made of 9 DNA base pairs) interposed; in pPαNE42, the 423rd valine codon was bound to the αNE gene, also with the same linker interposed; and in pPαNE43, the 374th aspartic acid codon was bound to the αNE gene, again with the interposition of the linker.

Claims:

1.  A plasmid which contains a promoter for the gene controlling a repressible yeast acid phosphatase and a gene for target peptide and which is capable of inducively expressing the gene for the target peptide in the yeast.

2.  A process for producing the target peptide which comprises preparing a plasmid bearing a promoter for the gene controlling a repressible yeast acid phosphatase and a gene for the target peptide, transforming yeast with said plasmid, and culturing the transformed yeast under inducible conditions.

# Fig. 1

Ba : BamHI, Hf : Hinfl, Kp : KpnI , Ac : AccI, Sa : SalI,

HII : HpaII , HI : HpaI

# Fig. 2a

```
         -210          -200          -190          -180          -170          -160          -150          -140
5'-CTGGTCCCTG TTTTCGAAGA GATCGCACAT GCCAAATTAT CAAATTGGTC ACCTTACTTG GCAAGGCATA TACCCATTTG
3'-GACCAGGGAC AAAAGCTTCT CTAGCGTGTA CGGTTTAATA GTTTAACCAG TGGAATGAAC CCTTCCGTAT ATGGGTAAAC
         -130          -120          -110          -100           -90           -80           -70           -60
  GGATAAGGGT AAACATCTTT GAATTGTCGA AATGAAACGT ATATAAGCGC TGATGTTTTG CTAAGTCGAG GTTAGTATGGC
  CCTATTCCCA TTTGTAGAAA CTTAACAGCT TTACTTTGCA TATATTCGCG ACTACAAAAC GATTCAGCTC CAATCATACCG
          -50           -40           -30           -20           -10          -17
  TTCATCTCT CATGAGAATA AGAACAACAA CAAATAGAGC AAGCAAATTC GAGATTACCA ATG TTT AAA TCT GTT GTT
  AAGTAGAGA GTACTCTTAT TCTTGTTGTT GTTTATCTCG TTCGTTTAAG CTCTAATGGT Met Phe Lys Ser Val Val
         -10                         -5                         -1    1                         5                          10
  TAT TCA ATT TTA GCC GCT TCT TTG GCC AAT GCA GGT ACC ATT CCC TTA GGC AAA CTA GCC GAT GTC
  Tyr Ser Ile Leu Ala Ala Ser leu Ala Asn Ala Gly Thr Ile Pro Leu Gly Lys Leu Ala Asp Val
                        15                          20                          25                          30
  GAC AAG ATT GGT ACC CAA AAA TAT ATC TTC CCA TTT TTG GGT GGT GCC GGA CCA TAC TAC TCT TTC
  Asp Lys Ile Gly Thr Gln Lys Tyr Ile Phe Pro Phe Leu Gly Gly Ala Gly Pro Tyr Tyr Ser Phe
          35                          40                          45                          50                          55
  CAT GGC GAC TAT GGT ATT TCT CGT GAT TTT CCT GAA GGT TGT GAA ATG AAG CAA CTG CAA ATG GTT
  His Gly Asp Tyr Gly Ile Ser Arg Asp Leu Pro Glu Gly Cys Glu Met Lys Gln Leu Gln Met Val
                        60                          65                          70                          75
  GGT AGG CAT GGT GAA AGA TAC CCT ACT GTC AGT CTG GCT AAG ACT ATC AAG AGT ACA TGG TAT AAG
  Gly Arg His Gly Glu Arg Tyr Pro Thr Val Ser Leu Ala Lys Thr Ile Lys Ser Thr Trp Tyr Lys
               80                          85                          90                          95
  TTG AGC AAT TAC ACT CGT CAA TTC AAC GGC TCA TTG TCA TTC TTG AAC GAT GAT TAC GAG TTT TTC
  Leu Ser Asn Tyr Thr Arg Gln Phe Asn Gly Ser Leu Ser Phe Leu Asn Asp Asp Tyr Glu Phe Phe
```

## Fig. 2b

```
100                    105                  110                    115                    120
ATC CGT GAT GAC GAT GAT TTG GAA ATG GAA ACC ACT TTT GGG AAC TCG GAC GAT GTT TTG AAC CCA
Ile Arg Asp Asp Asp Asp Leu Glu Met Glu Thr Thr Phe Gly Asn Ser Asp Asp Val Leu Asn Pro

            125                  130                    135                    140
TAC ACT GGT GAA ATG AAC GCC AAG AGA CAT GCT CGT GAC TTC TTG GCT CAA TAC GGT TAC ATG GTC
Tyr Thr Gly Glu Met Asn Ala Lys Arg His Ala Arg Asp Phe Leu Ala Gln Tyr Gly Tyr Met Val

      145                  150                    155                    160                    165
GAA AAC CAA ACC AGT TTC GCC GTT TTT ACC TCT AAT TCT AAG AGA TGT CAT GAC ACT GCT CAA TAT
Glu Asn Gln Thr Ser Phe Ala Val Phe Thr Ser Asn Ser Lys Arg Cys His Asp Thr Ala Cln Tyr

                  170                    175                    180                    185
TTC ATT CAT GGT TTA GGT GAC CAA TTC AAC ATC ACC TTG CAG ACT GTC AGT GAA GCT GAA TCC GCT
Phe Ile Asp Gly Leu Gly Asp Gln Phe Asn Ile Thr Leu Gln Thr Val Ser Glu Ala Glu Ser Ale

            190                  195                    200                    205
GGT GCC AAC ACT TTG AGT GCT TGT AAC TCA TGT CCT GCC TGG GAC TAC GAT GCC AAT GAT GAC ATT
Gly Ala Asn Thr Leu Ser Ala Cys Asn Ser Cys Pro Ala Trp Asp Tyr Asp Ala Asn Asp Asp Ile

210                    215                    220                    225                    230
GTA AAT GAA TAC GAC ACA ACC TAC TTG GAT CAC ATT CCC AAG AGA TTG AAC AAG GAA AAC AAG GGT
Val Asn Glu Tyr Asp Thr Thr Tyr Leu Asp Asp Ile Ala Lys Arg Leu Asn Lys Glu Asn Lys Gly

            235                  240                    245                    250
TTG AAC TTG ACC TCA ACT GAC GCT AGT ACT TTA TTC TCG TGG TGT GCA TTT GAA GTG AAC GCC AAA
Leu Asn Leu Thr Ser Thr Asp Ala Ser Thr Leu Phe Ser Trp Cys Ala Phe Glu Val Asn Ala Lys

      255                  260                    265                    270                    275
GGT TAC AGT GAT GTC TGT GAT ATT TTC ACC AAG GAT GAA TTA GTC CAT TAC TCC TAC TAC CAA GAC
Gly Tyr Ser Asp Val Cys Asp Ile Phe Thr Lys Asp Glu Leu Val His Tyr Ser Tyr Tyr Gln Asp
```

3

0109560

# Fig. 2c

```
                    280                    285                    290                    295
TTG CAG ACC TAT TAC CAT GAA GGT CCA GGT TAC GAT ATT ATC AAG TCT GTC GGT TCC AAC TTG TTC
Leu Gln Thr Tyr Tyr His Glu Gly Pro Gly Tyr Asp Ile Ile Lys Ser Val Gly Ser Asn Leu Phe

            300                    305                    310                    315
AAT GCC TCA GTC AAA TTA TTA AAG CAA AGT GAG ATT CAA GAC CAA AAG CTT TGG TTG AGT TTT ACC
Asn Ala Ser Val Lys Leu Leu Lys Gln Ser Glu Ile Gln Asp Gln Lys Val Trp Leu Ser Phe Thr

320                    325                    330                    335                    340
CAC GAT ACC GAT ATC CTA AAC TTT TTG ACC ACC GCT GGT ATA ATT GAC GAC AAA AAC AAC TTA ACT
His Asp Thr Asp Ile Leu Asn Phe Leu Thr Thr Ala Gly Ile Ile Asp Asp Lys Asn Asn Leu Thr

            345                    350                    355                    360
GCC GAA TAC GTT CCA TTC ATG GGC AAC ACT TTC CAC AGA TCC TGG TAC GTT CCT CAA GGT GCT CGT
Ala Glu Tyr Val Pro Phe Met Gly Asn Thr Phe His Arg Ser Trp Tyr Val Pro Gln Gly Ala Arg

    365                    370                    375                    380                    385
GTC TAC ACC GAA AAA TTC CAA TGT TCT AAC GAC ACC TAC GTC AGA TAC GTC ATT AAC GAT GCT GTC
Val Tyr Thr Glu lys Phe Gln Cys Ser Asn Asp Thr Tyr Val Arg Tyr Val Ile Asn Asp Ala Val

                    390                    395                    400                    405
GTT CCA ATT GAA ACC TGT TCC ACT GGT CCA GGG TTC TCT TGT GAA ATC AAT GAC TTC TAC GAC TAT
Val Pro Ile Glu Thr Cys Ser Thr Gly Pro Gly Phe Ser Cys Glu Ile Asn Asp Phe Tyr Asp Tyr

            410                    415                    420                    425
GCT GAA AAG AGA GTA GCC GGT ACT GAC TTC CTA AAG GTC TGT AAC GTC AGC AGT GTC AGT AAC GTC
Ala Glu Lys Arg Val Ala Gly Thr Asp Phe Leu Lys Val Cys Asn Val Ser Ser Val Ser Asn Val

430                    435                    440                    445                    450
ACC GAA TTG ACC TTC TAC TGG GAC TGG AAT ACT ACT CAC TAC AAT GAT ACC CTA TTA AAA CAA TAA
Thr Glu Leu Thr Phe Tyr Trp Asp Trp Asn Thr Thr His Tyr Asn Asp Thr Leu Leu Lys Gln Ocher
```

## Fig.2d

```
 +1        +10           +20           +30           +40           + 50          +60           +70           +80
ATTGTATAAA TAAATAATAT TGCAAATATA AATACCCATC GATTCTGATC TTCTTTTATG TACTAGTAGG GTAACGAGTT
TAACATATTT ATTTATTATA ACGTTTATAT TTATGGGTAG CTAAGACTAG AAGAAAATAC ATGATCATCC CATTGCTCAA
          +90          +100          +110          +120          +130          +140          +150          +160
TGTAGATTAG CGGCAGAATG AAAAGAAAAA AGTTAACTAA AAAGTAAGAA CAGCAAAGTT GATTGTAGCC TATATTGCTG
ACATCTAATC GCCGTCTTAC TTTTCTTTTT TCAATTGATT TTTCATTCTT GTCGTTTCAA CTAACATCGG ATATAACGAC
          +170          +180
AAATAGATTT CGGAGCTCCC GTATAAT-3'
TTTATCTAAA GCCTCGAGGG CATATTA-5'
```

6

# Fig. 3

*Fig. 4*

8

# Fig. 5